# EUROPEAN PATENT APPLICATION

(11) **EP 2 764 862 A1**
(43) Date of publication of application: **13.08.2014**
(21) Application number: 13154290.4
(22) Date of filing: 06.02.2013
(51) Int. Cl.: A61K 9/20, A61K 31/135, A61P 25/16

(54) **Immediate release tablets of rasagiline hemitartrate**

(71) Applicant: Galenicum Health S.L., 08005 Barcelona (ES)
(72) Inventor: Pérez Cadahía, Beatriz, 08005 Barcelona (ES)
(74) Representative: Torrejón-Nieto, Javier

(57) **Abstract**

The present invention relates to stable pharmaceutical compositions of (R)-N-propargyl-1-aminoindan hemitartrate in the form of immediate release tablets and to a process for the manufacture of said stable pharmaceutical compositions and of uniform pharmaceutical batches of said immediate release tablets.

## Description

The present invention relates to stable pharmaceutical compositions of (*R*)-N-propargyl-1-aminoindan hemitartrate in the form of immediate release tablets and to a process for the manufacture of said stable pharmaceutical compositions and of uniform pharmaceutical batches of said immediate release tablets.

### STATE OF THE ART

Parkinson's disease (also known as PD, Parkinson's, idiopathic parkinsonism, primary parkinsonism, hypokinetic rigid syndrome or *paralysis agitans*) is a degenerative disorder of the central nervous system. The motor symptoms of Parkinson's disease result from the death of dopamine-generating cells in the substantia nigra, a region of the midbrain. The cause of this cell death is unknown. Early in the course of the disease, the most obvious symptoms are movement-related, including shaking, rigidity, slowness of movement and difficulty with walking and gait. Later, cognitive and behavioural problems may arise, with dementia commonly occurring in the advanced stages of the disease. Other symptoms include sensory, sleep and emotional problems. Parkinson's disease is more common in the elderly, with most cases occurring after the age of 50.

Azilect® is a medicinal product indicated for the treatment of idiopathic Parkinson's disease as monotherapy (without levodopa) or as adjunct therapy (with levodopa) in patients with end of dose fluctuations. Azilect® contains (*R*)-N-propargyl-1-aminoindan mesylate as the active substance. It is presented as tablets each one containing an amount of (*R*)-N-propargyl-1-aminoindan mesylate equivalent to 1 mg of (*R*)-N-propargyl-1-aminoindan free base. Even using a aluminium/aluminium packaging, Azilect® requires that the storage temperature is not above 25°C.

(*R*)-N-propargyl-1-aminoindan is a selective irreversible inhibitor of the enzyme monoamine oxidase (MAO). (*R*)-N-propargyl-1-aminoindan was first disclosed in the EP0436492. Up to date, the only (*R*)-N-propargyl-1-aminoindan salt approved for use as a medicinal product has been the (*R*)-N-propargyl-1-aminoindan mesylate salt, which was first disclosed in the EP0812190. EP0812190 studies the solubility of several salts of (*R*)-N-propargyl-1-aminoindan and teaches that sulphate, esylate and mesylate salts possess significant advantages relative to the other salts due to good solubility and chemical stability, and that the mesylate salt is preferred. WO2011064216 discloses that the L-mandelate and (+)-camphor-10-sulfonate salts have good solubility. US8143315 mentions that it is essential to have the edisylate or oxalate salt of (*R*)-N-propargyl-1-aminoindan in order to have stable tablets.

### SUMMARY OF THE INVENTION

The present invention provides stable pharmaceutical compositions of *(R)*-N-propargyl-1-aminoindan hemitartrate in the form of immediate release tablets. The stable immediate release tablets as disclosed herein can be stored at temperatures higher than 25°C and are stable even when packaged in aluminium/PVC.

The stable immediate release tablets as disclosed herein are uniform in content even when manufactured by direct compression. The pharmaceutical batches of the pharmaceutical compositions of the present invention present content uniformity even when the active ingredient is in low concentration (even less than 1 % of the total weight of the pharmaceutical composition). Moreover, the batches show good flowability, especially when microcrystalline cellulose or trehalose are used as filler. The presence of (*R*)-N-propargyl-1-aminoindan hemitartrate in the mix with the filler or fillers affects the properties of said mix even being at a low concentration. Surprisingly, (*R*)-N-propargyl-1-aminoindan hemitartrate shows good flowability, which is especially better when microcrystalline cellulose (concretely, AVICEL PH-102) is used as filler. The high stability of the pharmaceutical compositions as disclosed herein, especially when comprising mannitol and/or trehalose, allows the packaging in aluminium/PVC and the storage at temperatures above 25°C.

In a first aspect, the present invention relates to a stable pharmaceutical composition in the form of immediate release tablets comprising (*R*)-N-propargyl-1-aminoindan hemitartrate.

In a second aspect, the present invention relates to the pharmaceutical composition of the first aspect for use in the treatment of Parkinson's disease, particularly for use in the treatment of idiopathic Parkinson's disease as monotherapy or as adjunct therapy with levodopa in patients with end of dose fluctuations.

In a third aspect, the present invention relates to (*R*)-N-propargyl-1-aminoindan hemitartrate crystalline form GH having at least X-ray powder diffraction peaks 6.7, 12.6 and 16.4° 2θ (±0.2° 2θ).

In a fourth aspect, the present invention relates to the use of the (*R*)-N-propargyl-1-aminoindan hemitartrate of the third aspect for the manufacture of immediate release tablets comprising (*R*)-N-propargyl-1-aminoindan hemitartrate.

In a fifth aspect, the present invention relates to the use of the (*R*)-N-propargyl-1-aminoindan hemitartrate of the third aspect for a process for the manufacture of immediate release tablets comprising (*R*)-N-propargyl-1-aminoindan hemitartrate.

In a sixth aspect, the present invention relates to a pharmaceutical batch comprising at least 20,000 units of the pharmaceutical composition of the first aspect of the present invention.

In a seventh aspect, the present invention relates to the pharmaceutical batch of the sixth aspect of the invention for use in the treatment of idiopathic Parkinson's disease as monotherapy or as adjunct therapy with levodopa in patients with end of dose fluctuations.

In an eighth aspect, the present invention relates to a process for the manufacture of the pharmaceutical composition of the first aspect or the pharmaceutical batch of the sixth aspect, wherein the process comprises the following steps:
i) sieving when appropriate the ingredients through a 500 micron sieve;
ii) mixing (*R*)-N-propargyl-1-aminoindan hemitartrate with at least one pharmaceutically acceptable excipient, preferably a filler, preferably at least 10 % of the total weight of said filler;
iii) further, and when appropriate, mixing the mixture obtained in step (ii) with further pharmaceutically acceptable excipients;
iv) optionally, mixing the mixture obtained in step (iii) or (ii) with at least one lubricant; and
v) compressing the final mix into tablets.

In a ninth aspect, the present invention relates to the pharmaceutical composition or the pharmaceutical batch obtained by the process of the eighth aspect of the invention.

In a tenth aspect, the present invention relates to the pharmaceutical batch of the sixth and ninth aspects of the invention for use in the treatment of Parkinson's Disease.

In an eleventh aspect, the present invention relates to a process for the manufacture of a stable pharmaceutical composition comprising (*R*)-N-propargyl-1-aminoindan hemitartrate in the form of an immediate release tablet, wherein the process comprises the following steps:
i) preparing a test pharmaceutical composition comprising (*R*)-N-propargyl-1-aminoindan hemitartrate in the form of a tablet;
ii) checking the stability and dissolution profile of the test pharmaceutical composition of step (i); and
iii) manufacturing a pharmaceutical composition comprising (R)-N-propargyl-1-aminoindan hemitartrate in the form of an immediate release tablet by the same process used to prepare the test pharmaceutical composition in step (i) provided that the test composition is stable and has an immediate release dissolution profile; and
iv) optionally, packaging the stable pharmaceutical composition manufactured in step (iii), preferably in a blister pack or a bottle.

In a twelfth aspect, the present invention relates to a process for the manufacture of a pharmaceutical batch of a pharmaceutical composition comprising *(R)*-N-propargyl-1-aminoindan hemitartrate in the form of an immediate release tablet, wherein the process comprises the following steps:
i) preparing a test pharmaceutical composition comprising (*R*)-N-propargyl-1-aminoindan hemitartrate in the form of a tablet;
ii) checking the stability and dissolution profile of the test pharmaceutical composition of step (i); and
iii) manufacturing a pharmaceutical batch by the same manufacturing process used to prepare the test pharmaceutical composition in step (i) provided that the test composition is stable and has an immediate release dissolution profile; and
iv) optionally, packaging the pharmaceutical batch manufactured in step (iii) preferably in blister packs or in bottles.

In a thirteenth aspect, the present invention relates to a process for the validation of a pharmaceutical batch of the sixth aspect of the invention, comprising the following steps:
i) manufacturing the pharmaceutical batch;
ii) checking the uniformity of the (*R*)-N-propargyl-1-aminoindan hemitartrate content; and
iii) validating the batch only if the content is uniform.

In a fourteenth aspect, the present invention relates to a pharmaceutical batch validated by the process of the thirteenth aspect of the invention.

In a fifteenth aspect, the present invention relates to a blister pack comprising the pharmaceutical composition of the first or ninth aspects, wherein said blister pack is preferably an aluminium/PVC blister pack or an aluminium/aluminium blister pack.

In a sixteenth aspect, the present invention relates to a cardboard box with a patient information leaflet comprising at least one aluminium/aluminium or aluminium/PVC blister pack of at least 4 tablets of the first or ninth aspects of the invention.

In a seventeenth aspect, the present invention relates to cardboard box with a patient information leaflet comprising a bottle containing at least 10 tablets of the first or ninth aspect of the invention.

### DETAILED DESCRIPTION OF THE INVENTION

The present invention provides stable pharmaceutical compositions of *(R)*-N-propargyl-1-aminoindan hemitartrate in the form of immediate release tablets.

(*R*)-N-(prop-2-ynyl)-2,3-dihydro-1H-inden-1-amine is also known as N-propargyl-1(*R*)-aminoindan, or as (*R*)-N-propargyl-1-aminoindan, or as *R*(+)-N-propargyl-1-aminoindan, or as rasagiline. The empirical formula of *(R)*-N-(prop-2-ynyl)-2,3-dihydro-1H-inden-1-amine hemitartrate is C12H13N. ½[C4H6O6] and the compound has a molecular weight of 246.22. The structural formula of *(R)*-N-(prop-2-ynyl)-2,3-dihydro-1H-inden-1-amine hemitartrate is (I):

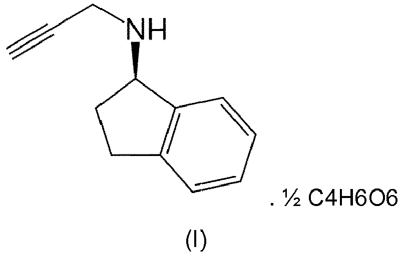

The term "stable" as used herein refers to a pharmaceutical composition comprising (*R*)-N-propargyl-1-aminoindan hemitartrate wherein the total content of impurities originating from the decomposition of (*R*)-N-propargyl-1-aminoindan hemitartrate does not exceed 5 % area, preferably 3 % area, more preferably 2 % area and most preferably 1 % area determined by liquid chromatography (HPLC) at 210 nm if such a composition is stored for 2 months at 40°C and 75 % relative humidity (RH).

An immediate release tablet as herein disclosed has to be understood as a tablet having a dissolution performance such as 60 % or more of the active agent contained in said pharmaceutical composition dissolves within 60 minutes (min). In a preferred embodiment, the immediate release composition as herein disclosed releases at least 80 % of the active agent in 60 minutes. In another embodiment, the pharmaceutical composition as herein disclosed releases at least 80 % of the active agent in 45 min, preferably in 35 min and more preferably in 30 min. In another embodiment, the pharmaceutical composition as herein disclosed releases at least 80 % of the active agent in 30 min and at least a 95 % of the active agent in 60 min. In a most preferred embodiment, the pharmaceutical composition as herein disclosed releases at least 80 % of the active agent in 15 min, and at least 95 % of the active agent in 60 min. The dissolution test for an immediate release pharmaceutical composition comprising the active agent as herein disclosed is performed in the following conditions: USP Apparatus: II (Paddles). Speed: 50 rpm. Medium: 0.1 N HCl pH 1.0. Wavelength 215 nm.

In a preferred embodiment of the pharmaceutical composition as herein disclosed, said pharmaceutical composition comprises between 0.05 and 10 % by weight of (*R*)-N-propargyl-1-aminoindan hemitartrate in respect of the total amount of the pharmaceutical composition. Preferably, it comprises between 0.1 and 5.0 % by weight of (*R*)-N-propargyl-1-aminoindan hemitartrate in respect of the total amount of the pharmaceutical composition. More preferably, it comprises between 0.2 and 3.0 % by weight of (*R*)-N-propargyl-1-aminoindan hemitartrate in respect of the total amount of the pharmaceutical composition. Even more preferably, it comprises between a 0.3 and a 1.5 % by weight of (*R*)-N-propargyl-1-aminoindan hemitartrate in respect of the total amount of the pharmaceutical composition.

In a preferred embodiment, the pharmaceutical composition comprises *(R)*-N-propargyl-1-aminoindan hemitartrate as the only active ingredient.

In a preferred embodiment of the pharmaceutical composition as herein disclosed, said pharmaceutical composition further comprises at least an acid. Said acid is a pharmaceutically acceptable acid. Preferably, the total amount of acid or acids present in the pharmaceutical composition ranges from 0.3 to 3.5 % by weight in respect of the total amount of the pharmaceutical composition. More preferably, the total amount of acid or acids present in the pharmaceutical composition ranges from 0.5 to 3 % by weight in respect of the total amount of the pharmaceutical composition. Even more preferably, the total amount of acid or acids present in the pharmaceutical composition ranges from 0.7 to 2.5 % by weight in respect of the total amount of the pharmaceutical composition.

High stability results were obtained for tablets as herein disclosed comprising microcrystalline cellulose, mannitol, trehalose or mixtures thereof and at least an acid. The stability results were especially good when the acid was citric acid or tartaric acid. The best results were obtained when the acid was tartaric acid.

In a preferred embodiment of the pharmaceutical composition as herein disclosed, said pharmaceutical composition comprises at least an acid selected from citric acid and tartaric acid. Preferably, the pharmaceutical composition comprises tartaric acid.

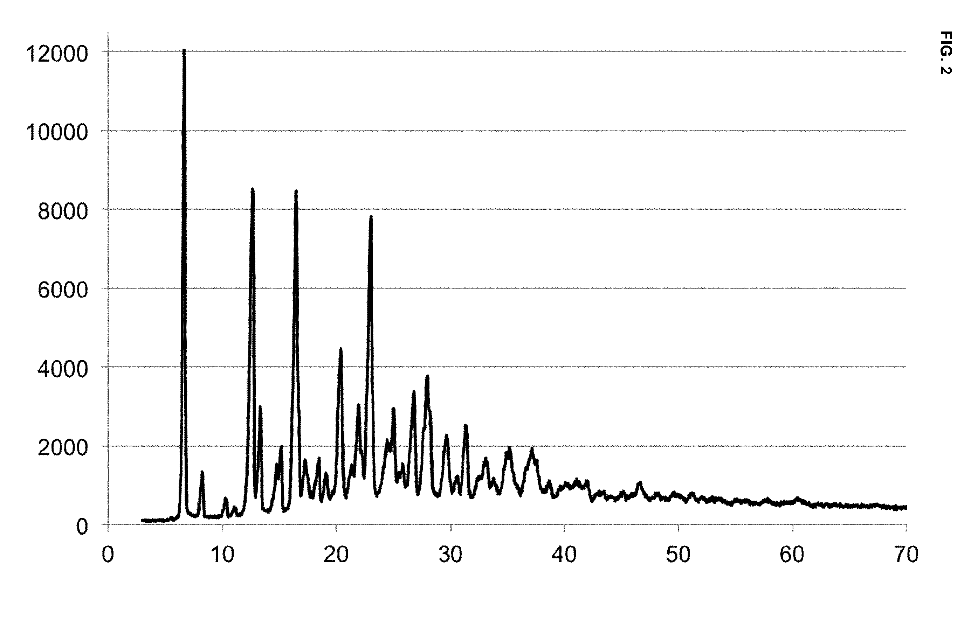

In a preferred embodiment of the pharmaceutical composition as herein disclosed, the (*R*)-N-propargyl-1-aminoindan hemitartrate is in crystalline form. Preferably, the (*R*)-N-propargyl-1-aminoindan hemitartrate comprises crystalline form GH characterised by at least X-ray powder diffraction peaks 6.7, 12.6 and 16.4° 2θ (±0.2° 2θ). Preferably, the (*R*)-N-propargyl-1-aminoindan hemitartrate consists of crystalline form GH characterised by at least X-ray powder diffraction peaks 6.7, 12.6 and 16.4° 2θ (±0.2° 2θ). More preferably, the (*R*)-N-propargyl-1-aminoindan hemitartrate comprises crystalline form GH characterised by at least X-ray powder diffraction peaks 6.7, 12.6, 13.3, 16.4, 20.4 and 23.0° 2θ (±0.2° 2θ). More preferably, the (*R*)-N-propargyl-1-aminoindan hemitartrate consists of crystalline form GH characterised by at least X-ray powder diffraction peaks 6.7, 12.6, 13.3, 16.4, 20.4 and 23.0° 2θ (±0.2° 2θ). More preferably, the (*R*)-N-propargyl-1-aminoindan hemitartrate has a X-ray powder diffraction pattern as depicted in figure 2. X-Ray Powder Diffraction was performed as explained in the examples.

When the pharmaceutical composition as herein disclosed comprises excipients such as a filler, an acid, a lubricant or a disintegrant, these excipients are pharmaceutically acceptable. The term "pharmaceutically acceptable" indicates that the substance or composition must be compatible chemically and/or toxicologically, with the other ingredients comprising a formulation, and/or the mammal being treated therewith.

In a preferred embodiment of the pharmaceutical composition as herein disclosed, said pharmaceutical composition comprises at least a filler. Preferably, the total amount of filler or fillers present in the pharmaceutical composition ranges from 25 % to 95 % by weight in respect of the total amount of the pharmaceutical composition. More preferably, the total amount of filler or fillers present in the pharmaceutical composition ranges from 45 % to 90 % by weight in respect of the total amount of the pharmaceutical composition. Even more preferably, the total amount of filler or fillers present in the pharmaceutical composition ranges from 60 % to 85 % by weight in respect of the total amount of the pharmaceutical composition.

The term "filler" as used herein refers to pharmaceutically acceptable excipients which are added to the bulk volume of the active agent making up the solid composition. As a result, the size of the solid composition increases, which makes its size suitable for handling. Fillers are convenient when the dose of drug per solid composition is low and the solid composition would otherwise be too small. In a preferred embodiment of the pharmaceutical composition as herein disclosed, said pharmaceutical composition comprises at least a filler selected from lactose, starch, mannitol, microcrystalline cellulose, trehalose, dibasic calcium phosphate, and mixtures thereof. Preferably, the pharmaceutical composition comprises at least a filler, selected from microcrystalline cellulose, mannitol and trehalose, and mixtures thereof, more preferably the pharmaceutical composition comprises trehalose. Preferably, the pharmaceutical composition comprises microcrystalline cellulose, trehalose and/or mannitol. Preferably, the pharmaceutical composition comprises microcrystalline cellulose. Preferably, the pharmaceutical composition comprises mannitol. The most preferred filler is trehalose.

As used herein, "lubricant" means a substance that reduces friction between the composition of the present invention and the surfaces of the apparatus used to compact the composition into a compressed form. In a preferred embodiment of the pharmaceutical composition as herein disclosed, said pharmaceutical composition comprises at least a lubricant. Preferably, the total amount of lubricant or lubricants present in the pharmaceutical composition ranges from 0.2 to 6 % by weight in respect of the total amount of the pharmaceutical composition. More preferably, the total amount of lubricant or lubricants present in the pharmaceutical composition ranges from 0.5 to 5 % by weight in respect of the total amount of the pharmaceutical composition. Even more preferably, the total amount of lubricant or lubricants present in the pharmaceutical composition ranges from 1 to 4 % by weight in respect of the total amount of the pharmaceutical composition.

In a preferred embodiment of the pharmaceutical composition as herein disclosed, said pharmaceutical composition comprises at least a lubricant selected from stearic acid, magnesium stearate, polyethylene glycol, sodium stearyl fumarate, sodium benzoate and mixtures thereof. Preferably, the pharmaceutical composition comprises at least a lubricant selected from magnesium stearate, stearic acid, sodium stearyl fumarate or mixtures thereof, more preferably the pharmaceutical composition comprises stearic acid.

As used herein, "disintegrant" means a substance or a mixture of substances added to a tablet to facilitate its breakup or disintegration after administration. In a preferred embodiment of the pharmaceutical composition as herein disclosed, said pharmaceutical composition comprises at least a disintegrant. Preferably, the total amount of disintegrant or disintegrants present in the pharmaceutical composition ranges from 5 to 25 % by weight in respect of the total amount of the pharmaceutical composition. More preferably, the total amount of disintegrant or disintegrants present in the pharmaceutical composition ranges from 8 to 22 % by weight in respect of the total amount of the pharmaceutical composition.

In a preferred embodiment of the pharmaceutical composition as herein disclosed, said pharmaceutical composition comprises at least a disintegrant selected from water-soluble disintegrants, such as starch, pregelatinized starch, sodium carboxymethyl cellulose, croscarmellose sodium, crosslinked polyvinylpyrrolidone, low-substituted hydroxypropyl cellulose and mixtures thereof. Preferably, the pharmaceutical composition comprises at least a disintegrant selected from starch, pregelatinized starch, and low-substituted hydroxypropyl cellulose, more preferably the pharmaceutical composition comprises pregelatinized starch.

In a preferred embodiment of the pharmaceutical composition as herein disclosed, the final water content of the pharmaceutical composition is from 0.2 % to 4 % by weight in respect of the total amount of the pharmaceutical composition. Preferably, the final water content of the pharmaceutical composition is from 0.3 % to 3.5 % by weight in respect of the total amount of the pharmaceutical composition.

In a preferred embodiment of the pharmaceutical composition as herein disclosed, the (*R*)-N-propargyl-1-aminoindan hemitartrate is not agglomerated. The term "agglomerated" as used herein refers to the active ingredient (*R*)-N-propargyl-1-aminoindan hemitartrate having an appearance of a crystalline powder with macroscopic agglomerates.

In a preferred embodiment of the pharmaceutical composition as herein disclosed, said pharmaceutical composition does not comprise citric acid or comprises citric acid in an amount other than 0.5 % to 2 % by weight in respect of the total amount of the pharmaceutical composition. In another preferred embodiment of the pharmaceutical composition as herein disclosed, said pharmaceutical composition is not the following: (*R*)-N-propargyl-1-aminoindan hemitartrate (equivalent to 1 mg of (*R*)-N-propargyl-1-aminoindan free base), 109.00 mg mannitol, 75.80 mg microcrystalline cellulose, 10.00 mg preagglutinated corn starch, 1.20 mg colloidal anhydrous silica, 2.00 mg citric acid, 2.00 mg magnesium stearate. In another preferred embodiment of the pharmaceutical composition as herein disclosed, said pharmaceutical composition is not the following: (*R*)-N-propargyl-1-aminoindan hemitartrate (equivalent to 1 mg of (*R*)-N-propargyl-1-aminoindan free base), 184.8 mg microcrystalline cellulose, 10.00 mg preagglutinated corn starch, 1.20 mg colloidal anhydrous silica, 2.00 mg citric acid, 2.00 mg magnesium stearate.

In a preferred embodiment of the pharmaceutical composition as herein disclosed, the D50 of (*R*)-N-propargyl-1-aminoindan hemitartrate is between 2 to 60 microns as measured by laser diffraction spectroscopy. Preferably, the D50 of *(R)*-N-propargyl-1-aminoindan hemitartrate is between 4 to 39 microns as measured by laser diffraction spectroscopy. More preferably, the D50 of (*R*)-N-propargyl-1-aminoindan hemitartrate is between 6 to 20 microns as measured by laser diffraction spectroscopy.

In a preferred embodiment of the pharmaceutical composition as herein disclosed, the D90 of (*R*)-N-propargyl-1-aminoindan hemitartrate is between 25 to 250 microns as measured by laser diffraction spectroscopy. Preferably, the D90 of *(R)*-N-propargyl-1-aminoindan hemitartrate is less than 159 microns as measured by laser diffraction spectroscopy. More preferably, the D90 of (*R*)-N-propargyl-1-aminoindan hemitartrate is between 35 to 80 microns as measured by laser diffraction spectroscopy.

In a preferred embodiment of the pharmaceutical composition as herein disclosed, the (*R*)-N-propargyl-1-aminoindan hemitartrate has a particle size other than D50 of 40.9 ± 0.57 microns, D90 of 161.2 ± 1.34 microns.

D50 and D90 represent the 50 percentile and the 90 percentile of the particle size volume distribution. That is, D50 (D90) is a value on the distribution such that 50 % (90 %) of the particles have a particle size of this value or less.

In a preferred embodiment of the pharmaceutical composition as herein disclosed, said pharmaceutical composition comprises between 0.74 and 2.18 mg of *(R)*-N-propargyl-1-aminoindan hemitartrate by unit dose. Preferably, it comprises about 1.44 mg of (*R*)-N-propargyl-1-aminoindan hemitartrate by unit dose.

In a preferred embodiment of the pharmaceutical composition as herein disclosed, said pharmaceutical compositions are manufactured by wet granulation techniques or dry mix techniques. Preferably, the pharmaceutical compositions are manufactured by direct compression.

In a preferred embodiment of the third aspect of the present invention, the *(R)*-N-propargyl-1-aminoindan hemitartrate has at least X-ray powder diffraction peaks 6.7, 12.6, 13.3, 16.4, 20.4 and 23.0° 2θ (±0.2° 2θ).

In a preferred embodiment of the pharmaceutical batch as herein disclosed, the *(R)-*N-propargyl-1-aminoindan hemitartrate content in the pharmaceutical compositions of the pharmaceutical batch is uniform.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active ingredient in the tablets of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active ingredient" refers to a therapeutically active compound, as well as any prodrugs thereof and pharmaceutically acceptable salts, hydrates and solvates of the compound and the prodrugs.

For the release of a pharmaceutical batch the distribution of the active ingredient in the tablets has to be homogeneous, that is, content uniformity is required.

All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

In a preferred embodiment of the pharmaceutical batch as herein disclosed, said pharmaceutical batch comprises at least 50,000 units, preferably comprises at least 100,000 units.

In a preferred embodiment, the tablets are packaged in a blister pack of aluminium/PVC or aluminium/aluminium. In a further embodiment of the pharmaceutical batch as herein disclosed, the pharmaceutical composition as herein disclosed are packaged in aluminium/PVC blisters. Although a blister aluminium/PVC is less protective than the aluminium/aluminium blister, the pharmaceutical composition as herein disclosed shows good stability in aluminum/PVC blisters, specially the one with mannitol and/or trehalose.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermomoldable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminium/PVC blister refers to a blister the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packing pharmaceutical products are the assurance of product/packaging integrity (including shelflife) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blisterline. There are two types of blister machine's design: rotary and flat-plate.

As used herein, the term "unit dose" or "unit dosage" refers to a physically discrete unit that contains a predetermined quantity of active ingredient calculated to produce a desired therapeutic effect. The unit dose or unit dosage may be in the form of a tablet, capsule, sachet, etc. referred to herein as a "unit dosage form."

In a preferred embodiment of both the eleventh and twelfth aspects of the present invention, the stability checked in step (ii) is the stability after at least one day at 40° C and 75 % relative humidity.

In a preferred embodiment of the sixteenth aspect of the present invention, the cardboard box comprises at least one aluminium/PVC blister pack of at least 4 tablets of the pharmaceutical composition as disclosed herein.

Further aspects and embodiments of the present invention can be found in the following numbered clauses:
Clause 1.- A stable pharmaceutical composition in the form of immediate release tablets comprising (*R*)-N-propargyl-1-aminoindan hemitartrate.
Clause 2.- The pharmaceutical composition according to the preceding clause comprising between 0.05 and 10 % by weight of (*R*)-N-propargyl-1-aminoindan hemitartrate in respect of the total amount of the pharmaceutical composition.
Clause 3.- The pharmaceutical composition according to any one of the preceding clauses comprising between 0.1 and 5.0 % by weight of (*R*)-N-propargyl-1-aminoindan hemitartrate in respect of the total amount of the pharmaceutical composition.
Clause 4.- The pharmaceutical composition according to any one of the preceding clauses comprising between 0.2 and 3.0 % by weight of (*R*)-N-propargyl-1-aminoindan hemitartrate in respect of the total amount of the pharmaceutical composition.
Clause 5.- The pharmaceutical composition according to any one of the preceding clauses comprising between a 0.3 and a 1.5 % by weight of (*R*)-N-propargyl-1-aminoindan hemitartrate in respect of the total amount of the pharmaceutical composition.
Clause 6.- The pharmaceutical composition according to any one of the preceding clauses, wherein the pharmaceutical composition further comprises at least an acid.
Clause 7.- The pharmaceutical composition according to the preceding clause, wherein the total amount of acid or acids present in the pharmaceutical composition ranges from 0.3 to 3.5 % by weight in respect of the total amount of the pharmaceutical composition.
Clause 8.- The pharmaceutical composition according to any one of the two preceding clauses, wherein the total amount of acid or acids present in the pharmaceutical composition ranges from 0.5 to 3 % by weight in respect of the total amount of the pharmaceutical composition.
Clause 9.- The pharmaceutical composition according to any one of the three preceding clauses, wherein the total amount of acid or acids present in the pharmaceutical composition ranges from 0.7 to 2.5 % by weight in respect of the total amount of the pharmaceutical composition.
Clause 10.- The pharmaceutical composition according to any one of the four preceding clauses, wherein the pharmaceutical composition comprises at least an acid selected from citric acid and tartaric acid.
Clause 11.- The pharmaceutical composition according to any one of the five preceding clauses, wherein the pharmaceutical composition comprises tartaric acid.
Clause 12.- The pharmaceutical composition according to any one of the preceding clauses, wherein the (*R*)-N-propargyl-1-aminoindan hemitartrate is in crystalline form.
Clause 13.- The pharmaceutical composition according to any one of the preceding clauses, wherein the (*R*)-N-propargyl-1-aminoindan hemitartrate comprises, preferably consists of, crystalline form GH characterised by at least X-ray powder diffraction peaks 6.7, 12.6 and 16.4° 2θ (±0.2° 2θ).
Clause 14.- The pharmaceutical composition according to the preceding clause, wherein the (*R*)-N-propargyl-1-aminoindan hemitartrate comprises, preferably consists of, crystalline form GH characterised by at least X-ray powder diffraction peaks 6.7, 12.6, 13.3, 16.4, 20.4 and 23.0° 2θ (±0.2° 2θ).
Clause 15.- The pharmaceutical composition according to any one of the preceding clauses, wherein the pharmaceutical composition comprises at least a filler.
Clause 16.- The pharmaceutical composition according to the preceding clause, wherein the total amount of filler or fillers present in the pharmaceutical composition ranges from 25 % to 95 % by weight in respect of the total amount of the pharmaceutical composition.
Clause 17.- The pharmaceutical composition according to any one of the two preceding clauses, wherein the total amount of filler or fillers present in the pharmaceutical composition ranges from 45 % to 90 % by weight in respect of the total amount of the pharmaceutical composition.
Clause 18.- The pharmaceutical composition according to any one of the three preceding clauses, wherein the total amount of filler or fillers present in the pharmaceutical composition ranges from 60 % to 85 % by weight in respect of the total amount of the pharmaceutical composition.
Clause 19.- The pharmaceutical composition according to any one of the four preceding clauses, wherein the pharmaceutical composition comprises at least a filler selected from lactose, starch, mannitol, microcrystalline cellulose, trehalose, dibasic calcium phosphate, and mixtures thereof.
Clause 20.- The pharmaceutical composition according to any one of the five preceding clauses, wherein the pharmaceutical composition comprises at least a filler, selected from microcrystalline cellulose, mannitol and trehalose, and mixtures thereof, preferably the pharmaceutical composition comprises trehalose; preferably the pharmaceutical composition comprises microcrystalline cellulose; preferably the pharmaceutical composition comprises mannitol. The most preferred filler is trehalose.
Clause 21.- The pharmaceutical composition according to any one of the preceding clauses, wherein the pharmaceutical composition comprises at least a lubricant.
Clause 22.- The pharmaceutical composition according to the preceding clause, wherein the total amount of lubricant or lubricants present in the pharmaceutical composition ranges from 0.2 to 6 % by weight in respect of the total amount of the pharmaceutical composition.
Clause 23.- The pharmaceutical composition according to any one of the two preceding clauses, wherein the total amount of lubricant or lubricants present in the pharmaceutical composition ranges from 0.5 to 5 % by weight in respect of the total amount of the pharmaceutical composition.
Clause 24.- The pharmaceutical composition according to any one of the three preceding clauses, wherein the total amount of lubricant or lubricants present in the pharmaceutical composition ranges from 1 to 4 % by weight in respect of the total amount of the pharmaceutical composition.
Clause 25.- The pharmaceutical composition according to any one of the four preceding clauses, wherein the pharmaceutical composition comprises at least a lubricant selected from stearic acid, magnesium stearate, polyethylene glycol, sodium stearyl fumarate, sodium benzoate and mixtures thereof.
Clause 26.- The pharmaceutical composition according to any one of the five preceding clauses, wherein the pharmaceutical composition comprises at least a lubricant selected from magnesium stearate, stearic acid, sodium stearyl fumarate or mixtures thereof, more preferably the pharmaceutical composition comprises stearic acid.
Clause 27.- The pharmaceutical composition according to any one of the preceding clauses, wherein the pharmaceutical composition comprises at least a disintegrant.
Clause 28.- The pharmaceutical composition according to the preceding clause, wherein the total amount of disintegrant or disintegrants present in the pharmaceutical composition ranges from 5 to 25 % by weight in respect of the total amount of the pharmaceutical composition.
Clause 29.- The pharmaceutical composition according to any one of the two preceding clauses, wherein the total amount of disintegrant or disintegrants present in the pharmaceutical composition ranges from 8 to 22 % by weight in respect of the total amount of the pharmaceutical composition.
Clause 30.- The pharmaceutical composition according to any one of the three preceding clauses, wherein the pharmaceutical composition comprises at least a disintegrant selected from starch, pregelatinized starch, sodium carboxymethyl cellulose, croscarmellose sodium, crosslinked polyvinylpyrrolidone, low-substituted hydroxypropyl cellulose and mixtures thereof.
Clause 31.- The pharmaceutical composition according to any one of the four preceding clauses, wherein the pharmaceutical composition comprises at least a disintegrant selected from starch, pregelatinized starch, and low-substituted hydroxypropyl cellulose, more preferably the pharmaceutical composition comprises pregelatinized starch.
Clause 32.- The pharmaceutical composition according to any one of the preceding clauses, wherein the final water content of the pharmaceutical composition is from 0.2 % to 4 % by weight in respect of the total amount of the pharmaceutical composition.
Clause 33.- The pharmaceutical composition according to the preceding clause, wherein the final water content of the pharmaceutical composition is from 0.3 % to 3.5 % by weight in respect of the total amount of the pharmaceutical composition.
Clause 34.- The pharmaceutical composition according to any one of the preceding clauses, wherein the (*R*)-N-propargyl-1-aminoindan hemitartrate is not agglomerated.
Clause 35.- The pharmaceutical composition according to any one of the preceding clauses, provided that the pharmaceutical composition does not comprise citric acid or comprises citric acid in an amount other than 0.5 % to 2 % by weight in respect of the total amount of the pharmaceutical composition.
Clause 36.- The pharmaceutical composition according to any one of the preceding clauses, provided that the pharmaceutical composition is not the following: *(R)*-N-propargyl-1-aminoindan hemitartrate (equivalent to 1 mg of (*R*)-N-propargyl-1-aminoindan free base), 109.00 mg mannitol, 75.80 mg microcrystalline cellulose, 10.00 mg preagglutinated corn starch, 1.20 mg colloidal anhydrous silica, 2.00 mg citric acid, 2.00 mg magnesium stearate.
Clause 37.- The pharmaceutical composition according to any one of the preceding clauses, provided that the pharmaceutical composition is not the following: *(R)*-N-propargyl-1-aminoindan hemitartrate (equivalent to 1 mg of (*R*)-N-propargyl-1-aminoindan free base), 184.8 mg microcrystalline cellulose, 10.00 mg preagglutinated corn starch, 1.20 mg colloidal anhydrous silica, 2.00 mg citric acid, 2.00 mg magnesium stearate.
Clause 38.- The pharmaceutical composition according to any one of the preceding clauses, wherein the D50 of (*R*)-N-propargyl-1-aminoindan hemitartrate is between 2 to 60 microns as measured by laser diffraction spectroscopy.
Clause 39.- The pharmaceutical composition according to the preceding clause, wherein the D50 of (*R*)-N-propargyl-1-aminoindan hemitartrate is between 4 to 39 microns as measured by laser diffraction spectroscopy.
Clause 40.- The pharmaceutical composition according to any one of the two preceding clauses, wherein the D50 of (*R*)-N-propargyl-1-aminoindan hemitartrate is between 6 to 20 microns as measured by laser diffraction spectroscopy.
Clause 41.- The pharmaceutical composition according to any one of the preceding clauses, wherein the D90 of (*R*)-N-propargyl-1-aminoindan hemitartrate is between 25 to 250 microns as measured by laser diffraction spectroscopy.
Clause 42.- The pharmaceutical composition according to any one of the preceding clauses, wherein the D90 of (*R*)-N-propargyl-1-aminoindan hemitartrate is less than 159 microns as measured by laser diffraction spectroscopy.
Clause 43.- The pharmaceutical composition according to the preceding clause, wherein the D90 of (*R*)-N-propargyl-1-aminoindan hemitartrate is between 35 to 80 microns as measured by laser diffraction spectroscopy.
Clause 44.- The pharmaceutical composition according to any one of the preceding clauses, provided that the (*R*)-N-propargyl-1-aminoindan hemitartrate has a particle size other than D50 of 40.9 ± 0.57 microns, D90 of 161.2 ± 1.34 microns.
Clause 45.- The pharmaceutical composition according to any one of the preceding clauses, wherein the pharmaceutical composition comprises between 0.74 and 2.18 mg of (*R*)-N-propargyl-1-aminoindan hemitartrate by unit dose.
Clause 46.- The pharmaceutical composition according to any one of the preceding clauses, wherein the pharmaceutical composition comprises about 1.44 mg of *(R)-*N-propargyl-1-aminoindan hemitartrate by unit dose.
Clause 47.- The pharmaceutical composition according to the preceding clause, wherein the pharmaceutical compositions are manufactured by wet granulation techniques or dry mix techniques.
Clause 48.- The pharmaceutical composition according to the preceding clause, wherein the pharmaceutical compositions are manufactured by direct compression.
Clause 49.- The pharmaceutical composition according to any one of the preceding clauses for use in the treatment of Parkinson's disease.
Clause 50.- The pharmaceutical composition according to any one of the preceding clauses for use in the treatment of idiopathic Parkinson's disease as monotherapy or as adjunct therapy with levodopa in patients with end of dose fluctuations.
Clause 51.- (*R*)-N-propargyl-1-aminoindan hemitartrate crystalline form GH having at least X-ray powder diffraction peaks 6.7, 12.6 and 16.4° 2θ (±0.2° 2θ).
Clause 52.- (*R*)-N-propargyl-1-aminoindan hemitartrate according to the preceding clause, having at least X-ray powder diffraction peaks 6.7, 12.6, 13.3, 16.4, 20.4 and 23.0° 2θ (±0.2° 2θ).
Clause 53.- Use of the (*R*)-N-propargyl-1-aminoindan hemitartrate according to any one of the two preceding clauses for the manufacture of immediate release tablets comprising (*R*)-N-propargyl-1-aminoindan hemitartrate.
Clause 54.- Use of the (*R*)-N-propargyl-1-aminoindan hemitartrate according to the preceding clause for a process for the manufacture of immediate release tablets comprising (*R*)-N-propargyl-1-aminoindan hemitartrate.
Clause 55.- A pharmaceutical batch comprising at least 20,000 units of the pharmaceutical composition as defined in any one of clauses 1 to 48.
Clause 56.- The pharmaceutical batch according to the preceding clause, wherein the (*R*)-N-propargyl-1-aminoindan hemitartrate content is uniform.
Clause 57.- The pharmaceutical batch according to any one of the two preceding clauses, comprising at least 50,000 units.
Clause 58.- The pharmaceutical batch according to any one of the three preceding clauses, comprising at least 100,000 units.
Clause 59.- The pharmaceutical batch according to any one of the four preceding clauses wherein the tablets are packaged in a blister pack of aluminium/PVC or aluminium/aluminium.
Clause 60.- The pharmaceutical batch according to any one of the five preceding clauses for use in the treatment of idiopathic Parkinson's disease as monotherapy or as adjunct therapy with levodopa in patients with end of dose fluctuations.
Clause 61.- A process for the manufacture of the pharmaceutical composition as defined in clauses 1 to 48 or the pharmaceutical batch as defined in clauses 55 to 60, wherein the process comprises the following steps:
   i) sieving when appropriate the ingredients through a 500 micron sieve;
   ii) mixing (*R*)-N-propargyl-1-aminoindan hemitartrate with at least one pharmaceutically acceptable excipient, preferably a filler, preferably at least 10 % of the total weight of said filler;
   iii) further, and when appropriate, mixing the mixture obtained in step (ii) with further pharmaceutically acceptable excipients;
   iv) optionally, mixing the mixture obtained in step (iii) or (ii) with at least one lubricant; and
   v) compressing the mix into tablets.
Clause 62.- The pharmaceutical composition or the pharmaceutical batch obtained by the process of the preceding clause.
Clause 63.- The pharmaceutical batch according to the preceding clause for use in the treatment of Parkinson's Disease.
Clause 64.- A process for the manufacture of a stable pharmaceutical composition comprising (*R*)-N-propargyl-1-aminoindan hemitartrate in the form of an immediate release tablet, wherein the process comprises the following steps:
   i) preparing a test pharmaceutical composition comprising *(R)*-N-propargyl-1-aminoindan hemitartrate in the form of a tablet;
   ii) checking the stability and dissolution profile of the test pharmaceutical composition of step (i); and
   iii) manufacturing a pharmaceutical composition comprising *(R)*-N-propargyl-1-aminoindan hemitartrate in the form of an immediate release tablet by the same process used to prepare the test pharmaceutical composition in step (i) provided that the test composition is stable and has an immediate release dissolution profile; and
   iv) optionally, packaging the stable pharmaceutical composition manufactured in step (iii), preferably in a blister pack or a bottle.
Clause 65.- A process for the manufacture of a pharmaceutical batch of a pharmaceutical composition comprising (*R*)-N-propargyl-1-aminoindan hemitartrate in the form of an immediate release tablet, wherein the process comprises the following steps:
   i) preparing a test pharmaceutical composition comprising *(R)*-N-propargyl-1-aminoindan hemitartrate in the form of a tablet;
   ii) checking the stability and dissolution profile of the test pharmaceutical composition of step (i); and
   iii) manufacturing a pharmaceutical batch by the same manufacturing process used to prepare the test pharmaceutical composition in step (i) provided that the test composition is stable and has an immediate release dissolution profile; and
   iv) optionally, packaging the pharmaceutical batch manufactured in step (iii) preferably in blister packs or in bottles.
Clause 66.- The process according to any of the two preceding clauses, wherein the stability checked in step (ii) is the stability after at least one day at 40°C and 75 % relative humidity.
Clause 67.- A process for the validation of a pharmaceutical batch as defined in any one of clauses 55 to 60, comprising the following steps:
   i) manufacturing the pharmaceutical batch;
   ii) checking the uniformity of the (*R*)-N-propargyl-1-aminoindan hemitartrate content; and
   iii) validating the batch only if the content is uniform.
Clause 68.- A pharmaceutical batch validated by the process of the preceding clause.
Clause 69.- A blister pack comprising the pharmaceutical composition as defined in any one of clauses 1 to 48 or in clause 62, wherein said blister pack is preferably an aluminium/PVC blister or an aluminium/aluminium blister.
Clause 70.- A cardboard box with a patient information leaflet comprising at least one aluminium/aluminium or aluminium/PVC blister pack of at least 4 tablets as defined in any one of the clauses 1 to 48 or in clause 62.
Clause 71.- A cardboard box with a patient information leaflet according to the preceding clause comprising at least one aluminium/PVC blister pack of at least 4 tablets as defined in any one of the clauses 1 to 48 or in clause 62.
Clause 72.- A cardboard box with a patient information leaflet comprising a bottle containing at least 10 tablets as defined in any one of the clauses 1 to 48. or in clause 62

Preferred combinations of the above clauses are as follows:

| | |
|---|---|
| Clause 73.- 1, 3 | Clause 105.- 1, 3, 42 |
| Clause 74.- 1, 7 | Clause 106.- 1, 7, 42 |
| Clause 75.- 1, 3, 7 | Clause 107.- 1, 3, 7, 42 |
| Clause 76.- 1, 16 | Clause 108.- 1, 16, 42 |
| Clause 77.- 1, 3, 16 | Clause 109.- 1, 3, 16, 42 |
| Clause 78.- 1, 7, 16 | Clause 110.- 1, 7, 16, 42 |
| Clause 79.- 1, 3, 7, 16 | Clause 111.- 1, 3, 7, 16, 42 |
| Clause 80.- 1, 20 | Clause 112.- 1, 20, 42 |
| Clause 81.- 1, 3, 20 | Clause 113.- 1, 3, 20, 42 |
| Clause 82.- 1, 7, 20 | Clause 114.- 1, 7, 20, 42 |
| Clause 83.- 1, 3, 7, 20 | Clause 115.- 1, 3, 7, 20, 42 |
| Clause 84.- 1, 16, 20 | Clause 116.- 1, 16, 20, 42 |
| Clause 85.- 1, 3, 16, 20 | Clause 117.- 1, 3, 16, 20, 42 |
| Clause 86.- 1, 7, 16, 20 | Clause 118.- 1, 7, 16, 20, 42 |
| Clause 87.- 1, 3, 7, 16, 20 | Clause 119.- 1, 3, 7, 16, 20, 42 |
| Clause 88.- 1, 39 | Clause 120.- 1, 39, 42 |
| Clause 89.- 1, 3, 39 | Clause 121.- 1, 3, 39, 42 |
| Clause 90.- 1, 7, 39 | Clause 122.- 1, 7, 39, 42 |
| Clause 91.- 1, 3, 7, 39 | Clause 123.- 1, 3, 7, 39, 42 |
| Clause 92.- 1, 16, 39 | Clause 124.- 1, 16, 39, 42 |
| Clause 93.- 1, 3, 16, 39 | Clause 125.- 1, 3, 16, 39, 42 |
| Clause 94.- 1, 7, 16, 39 | Clause 126.- 1, 7, 16, 39, 42 |
| Clause 95.- 1, 3, 7, 16, 39 | Clause 127.- 1, 3, 7, 16, 39, 42 |
| Clause 96.- 1, 20, 39 | Clause 128.- 1, 20, 39, 42 |
| Clause 97.- 1, 3, 20, 39 | Clause 129.- 1, 3, 20, 39, 42 |
| Clause 98.- 1, 7, 20, 39 | Clause 130.- 1, 7, 20, 39, 42 |
| Clause 99.- 1, 3, 7, 20, 39 | Clause 131.- 1, 3, 7, 20, 39, 42 |
| Clause 100.- 1, 16, 20, 39 | Clause 132.- 1, 16, 20, 39, 42 |
| Clause 101.- 1, 3, 16, 20, 39 | Clause 133.- 1, 3, 16, 20, 39, 42 |
| Clause 102.- 1, 7, 16, 20, 39 | Clause 134.- 1, 7, 16, 20, 39, 42 |
| Clause 103.- 1, 3, 7, 16, 20, 39 | Clause 135.- 1, 3, 7, 16, 20, 39, 42 |
| Clause 104.- 1, 42 | Clause 136.- 1, 11, 20 |

Clause 137.- A pharmaceutical composition according to any one of the clauses 73 to 136 packaged in an aluminium/aluminium or aluminium/PVC blister pack.
Clause 138.- A cardboard box with a patient information leaflet comprising the pharmaceutical composition according to any one of the clauses 73 to 136 packaged in an aluminium/aluminium or aluminium/PVC blister pack or in a bottle.

All percentages, parts, and ratios herein are by weight unless specifically noted otherwise. As used herein, the term "about" refers preferably to a range that is ±10 %, preferably ±5 %, or more preferably ±1 % of a value with which the term is associated.

### DESCRIPTION OF THE FIGURES

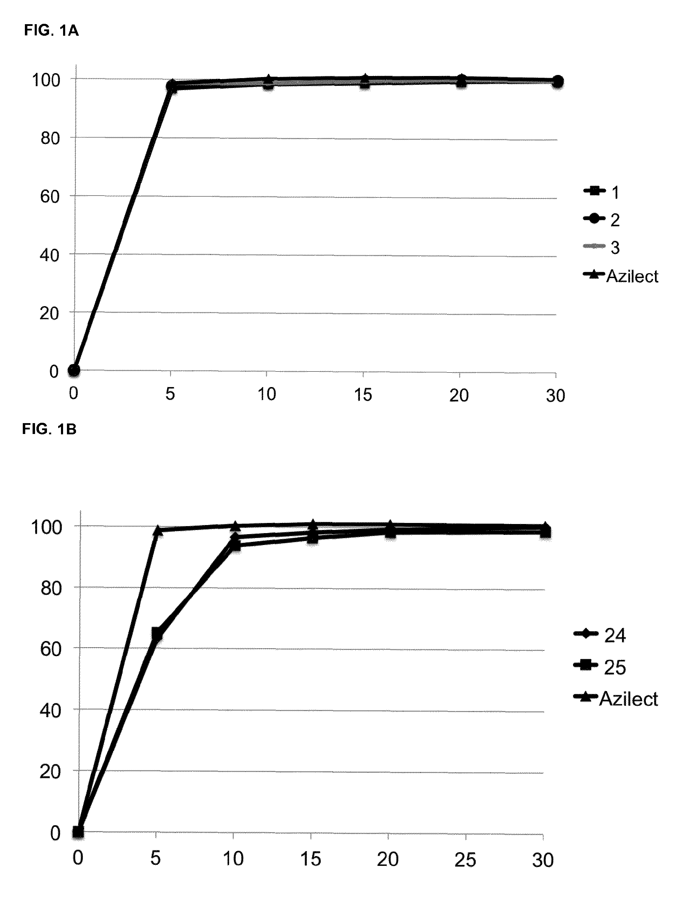

FIG. 1: Immediate release dissolution profiles. A. Percentage of the active agent dissolved over time in minutes for batches 1 to 3 of the present invention and Azilect, at pH 1.0. B. Percentage of the active agent dissolved over time in minutes for batches 24 and 25 of the present invention and Azilect, at pH 1.0.
FIG. 2: X-ray powder diffraction pattern (XRD) of (*R*)-N-propargyl-1-aminoindan hemitartrate crystalline form GH.
FIG. 3: Average particle size distribution. Horizontal axis: particle size in micrometers. Vertical axis: volume (%), indicating the percentage of particles with the corresponding particle size.

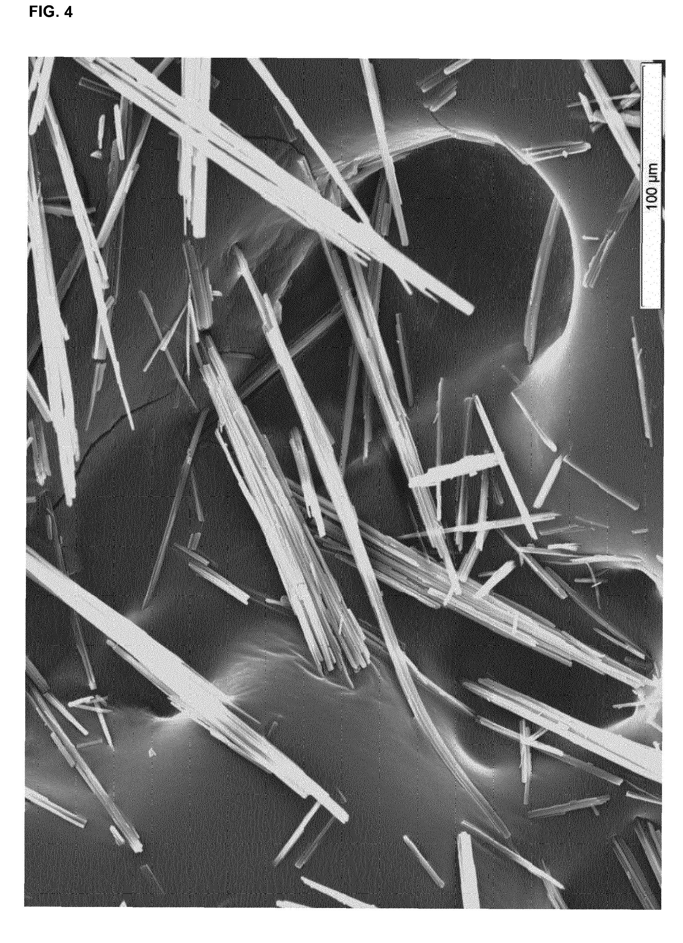

FIG. 4: Photograph of the (*R*)-N-propargyl-1-aminoindan hemitartrate by scanning electron microscopy.

### EXAMPLES

### Examples 1 to 3: (R)-N-propargyl-1-aminoindan hemitartrate tablet composition

| Examples | 1 | 2 | 3 |
|---|---|---|---|
| (*R*)-N-propargyl-1-aminoindan hemitartrate (mg) | 1.44 * | 1.44 * | 1.44 * |
| Mannitol (mg) | 154.8 | 156.9 | - |
| Microcrystalline cellulose (mg) | - | - | 154.8 |
| Pregelatinized starch (mg) | 21.0 | 21.0 | 21.0 |
| Starch (mg) | 21.0 | 21.0 | 21.0 |
| Colloidal silicon dioxide (mg) | 1.26 | 1.26 | 1.26 |
| Citric acid (mg) | 2.1 | - | 2.1 |
| Stearic acid (mg) | 4.2 | 4.2 | 4.2 |
| Talc USP (mg) | 4.2 | 4.2 | 4.2 |
| Total (mg/tablet) | 210.0 | 210.0 | 210.0 |

| | | | |
|---|---|---|---|
| * Equivalent to 1 mg (*R*)-N-propargyl-1-aminoindan free. | | | |

### Examples 4 to 6: (R)-N-propargyl-1-aminoindan hemitartrate tablet composition

| Examples | 4 | 5 | 6 |
|---|---|---|---|
| (*R*)-N-propargyl-1-aminoindan hemitartrate (mg) | 1.44 * | 1.44 * | 1.44 * |
| Mannitol (mg) | 161.1 | 159.0 | 150.6 |
| Pregelatinized starch (mg) | 21.0 | 21.0 | 21.0 |
| Starch (mg) | 21.0 | 21.0 | 21.0 |
| Colloidal silicon dioxide (mg) | 1.26 | 1.26 | 1.26 |
| Citric acid (mg) | - | 2.1 | 10.5 |
| Talc USP (mg) | 4.2 | 4.2 | 4.2 |
| Total (mg/tablet) | 210.0 | 210.0 | 210.0 |

| | | | |
|---|---|---|---|
| * Equivalent to 1 mg (*R*)-N-propargyl-1-aminoindan free base. | | | |

### Examples 7 to 9: (R)-N-propargyl-1-aminoindan hemitartrate tablet composition

| Examples | 7 | 8 | 9 |
|---|---|---|---|
| (*R*)-N-propargyl-1-aminoindan hemitartrate (mg) | 1.44 * | 1.44 * | 1.44 * |
| Mannitol (mg) | 159.0 | 174.3 | 172.2 |
| Pregelatinized starch (mg) | 21.0 | 12.3 | 12.3 |
| Starch (mg) | 21.0 | 12.3 | 12.3 |
| Colloidal silicon dioxide (mg) | 1.26 | 1.26 | 1.26 |
| Tartaric acid (mg) | 2.1 | - | - |
| Citric acid (mg) | - | - | 2.1 |
| Stearic acid (mg) | 4.2 | 4.2 | 4.2 |
| Talc USP (mg) | 4.2 | 4.2 | 4.2 |
| Total (mg/tablet) | 210.0 | 210.0 | 210.0 |

| | | | |
|---|---|---|---|
| * Equivalent to 1 mg (*R*)-N-propargyl-1-aminoindan free base. | | | |

### Examples 10 to 12: (R)-N-propargyl-1-aminoindan hemitartrate tablet composition

| Examples | 10 | 11 | 12 |
|---|---|---|---|
| (*R*)-N-propargyl-1-aminoindan hemitartrate (mg) | 1.44 * | 1.44 * | 1.44 * |
| Mannitol (mg) | 172.2 | 172.2 | 172.2 |
| Pregelatinized starch (mg) | 12.3 | 12.3 | 12.3 |
| Starch (mg) | 12.3 | 12.3 | 12.3 |
| Colloidal silicon dioxide (mg) | 1.26 | 1.26 | 1.26 |
| Tartaric acid (mg) | 2.1 | 2.1 | 2.1 |
| Stearic acid (mg) | 4.2 | - | 4.2 |
| Magnesium stearate (mg) | - | 4.2 | - |
| Sodium stearyl fumarate (mg) | - | - | 4.2 |
| Talc USP (mg) | 4.2 | 4.2 | 4.2 |
| Total (mg/tablet) | 210.0 | 210.0 | 214.2 |

| | | | |
|---|---|---|---|
| * Equivalent to 1 mg (*R*)-N-propargyl-1-aminoindan free base. | | | |

### Examples 13 to 15: (R)-N-propargyl-1-aminoindan hemitartrate tablet composition

| Examples | 13 | 14 | 15 |
|---|---|---|---|
| (*R*)-N-propargyl-1-aminoindan hemitartrate (mg) | 1.44* | 1.44* | 1.44* |
| Mannitol (mg) | 172.2 | 172.2 | 172.2 |
| Low-substituted hydroxypropyl cellulose (mg) | 12.3 | 12.3 | 12.3 |
| Starch (mg) | 12.3 | 12.3 | 12.3 |
| Colloidal silicon dioxide (mg) | 1.26 | 1.26 | 1.26 |
| Tartaric acid (mg) | 2.1 | 2.1 | 2.1 |
| Stearic acid (mg) | 4.2 | 4.2 | 8.4 |
| Sodium stearyl fumarate (mg) | 4.2 | - | - |
| Talc USP (mg) | 4.2 | 4.2 | 8.4 |
| Total (mg/tablet) | 214.2 | 210.0 | 218.4 |

| | | | |
|---|---|---|---|
| * Equivalent to 1 mg (*R*)-N-propargyl-1-aminoindan free base. | | | |

### Examples 16/17: (R)-N-propargyl-1-aminoindan hemitartrate tablet composition

| Examples | 16/17 |
|---|---|
| (*R*)-N-propargyl-1-aminoindan hemitartrate (mg) | 1.44* |
| Mannitol (mg) | 176.40 |
| Pregelatinized starch (mg) | 12.3 |
| Starch (mg) | 12.3 |
| Colloidal silicon dioxide (mg) | 1.26 |
| Tartaric acid (mg) | 2.1 |
| Stearic acid (mg) | 4.2 |
| Talc USP (mg) | 4.2 |
| Total (mg/tablet) | 214.2 |

| | |
|---|---|
| * Equivalent to 1 mg (*R*)-N-propargyl-1-aminoindan free base. | |

### Examples 18 and 19: (R)-N-propargyl-1-aminoindan hemitartrate tablet composition

| Examples | 18 | 19 |
|---|---|---|
| (*R*)-N-propargyl-1-aminoindan hemitartrate (mg) | 1.44* | 1.44* |
| Mannitol (mg) | 172.2 | 172.2 |
| Low-substituted hydroxypropyl cellulose (mg) | 12.3 | - |
| Pregelatinized starch (mg) | - | 12.3 |
| Starch (mg) | 12.3 | 12.3 |
| Colloidal silicon dioxide (mg) | 1.26 | 1.26 |
| Tartaric acid (mg) | 2.1 | 2.1 |
| Stearic acid (mg) | 4.2 | - |
| Sodium stearyl fumarate (mg) | 4.2 | - |
| Magnesium stearate (mg) | - | 4.2 |
| Talc USP (mg) | 4.2 | 4.2 |
| Total (mg/tablet) | 216.3 | 210.0 |

| | | |
|---|---|---|
| * Equivalent to 1 mg (*R*)-N-propargyl-1-aminoindan free base. | | |

### Examples 20 and 21: (R)-N-propargyl-1-aminoindan hemitartrate tablet composition

| Examples | 20 | 21 |
|---|---|---|
| (*R*)-N-propargyl-1-aminoindan hemitartrate (mg) | 1.44* | 1.44* |
| Microcrystalline cellulose (mg) | 154.8 | 154.8 |
| Pregelatinized starch (mg) | 21.0 | 21.0 |
| Starch (mg) | 21.0 | 21.0 |
| Colloidal silicon dioxide (mg) | 1.26 | 1.26 |
| Tartaric acid (mg) | 2.1 | - |
| Stearic acid (mg) | 4.2 | 4.2 |
| Talc USP (mg) | 4.2 | 4.2 |
| Total (mg/tablet) | 210.0 | 207.9 |

| | | |
|---|---|---|
| * Equivalent to 1 mg (*R*)-N-propargyl-1-aminoindan free base. | | |

### Examples 22 and 23: (R)-N-propargyl-1-aminoindan hemitartrate tablet composition

| Examples | 22 | 23 |
|---|---|---|
| (*R*)-N-propargyl-1-aminoindan hemitartrate (mg) | 1.44* | 1.44* |
| Microcrystalline cellulose (mg) | 154.8 | 154.8 |
| Pregelatinized starch (mg) | 21.0 | 21.0 |
| Starch (mg) | 21.0 | - |
| Anhydrous dicalcium phosphate (mg) | - | 21.0 |
| Colloidal silicon dioxide (mg) | 1.26 | 1.26 |
| Tartaric acid (mg) | 4.2 | 2.1 |
| Stearic acid (mg) | 4.2 | 4.2 |
| Talc USP (mg) | 4.2 | 4.2 |
| Total (mg/tablet) | 210.0 | 210.0 |

| | | |
|---|---|---|
| * Equivalent to 1 mg (*R*)-N-propargyl-1-aminoindan free base. | | |

### Examples 24 and 25: (R)-N-propargyl-1-aminoindan hemitartrate tablet composition

| Examples | 24 | 25 |
|---|---|---|
| (*R*)-N-propargyl-1-aminoindan hemitartrate (mg) | 1.44* | 1.44* |
| Trehalose (mg) | 175.8 | 154.8 |
| Pregelatinized starch (mg) | 21.0 | 21.0 |
| Microcrystalline cellulose (mg) | - | 21.0 |
| Colloidal silicon dioxide (mg) | 1.26 | 1.26 |
| Tartaric acid (mg) | 2.1 | 2.1 |
| Stearic acid (mg) | 4.2 | 4.2 |
| Talc USP (mg) | 4.2 | 4.2 |
| Total (mg/tablet) | 210.0 | 210.0 |

| | | |
|---|---|---|
| * Equivalent to 1 mg (*R*)-N-propargyl-1-aminoindan free base. | | |

### Example 26: Batches manufactured by direct dry mix

All ingredients were sifted through a 0.5 mm sieve. The (*R*)-N-propargyl-1-aminoindan hemitartrate was mixed with around 15 % of the filler during 10 min at 34 rpm. The rest of the filler was added to the mixture and mixed during 10 min at 34 rpm. The rest of the excipients except for the lubricant were added and mixed during 10 min at 34 rpm. The lubricant was added and mixed during 5 min at 34 rpm. The dry mix was then compressed into tablets using a compression force of about 80 N. Batches of 500 to 5,000 tablets were manufactured for the compositions of examples 1 to 25. These batches are referred to as batches 1 to 25.

### Example 27: Content uniformity

The (*R*)-N-propargyl-1-aminoindan hemitartrate content and the amount of impurities were analysed by HPLC. The tablets were finely powdered and extracted with a diluent such as a mixture of water and acetonitrile. A sample was injected into an HPLC and eluted using the same diluent. The area corresponding to the *(R)*-N-propargyl-1-aminoindan hemitartrate and of the major peaks, if any, was determined. The percent of impurities was calculated by comparing the areas of the measured peaks with those obtained from the standard.

The content uniformity was tested for batches 8, 9, 10, 16 and 17. In all cases, the content uniformity was above 90 % and the RSD below 2 %. For example, in batch 16, the average content of (*R*)-N-propargyl-1-aminoindan hemitartrate was 97.1 % and the RSD of 1.33 % and in batch 11, the average content of (*R*)-N-propargyl-1-aminoindan hemitartrate was 99.1 % and the RSD of 0.11 %.

### Example 28: Stability

Tablets of examples 1 to 25 were packaged in bottles or in blisters packs of aluminium/PVC or aluminium/aluminium. Tablets of examples 1 to 25 as well as tablets of the marketed product Azilect were subjected to stability tests at times 0 and 72 hours, at 40°C and 75 % Relative Humidity (RH); 50°C and saturated RH and 70°C (Accelerated stability tests) or at times 0, 30 days (1 month) and 60 days (2 months) at 25°C and 60 % RH, 30°C and 65 % RH and 40°C and 75 % RH (Accelerated ICH stability tests).

The stability results of batches 20 to 23 showed that these formulations are suitable for being marketed.

**TABLE A. Accelerated stability tests (72 hours). Percentage of total impurities.**

| **Batch** | **t = 0** | **40°C/75 % RH** |
|---|---|---|
| Azilect | 0.13 | 0.12 |
| 1 | 0.09 | 0.09 |
| 2 | 0.08 | 0.12 |
| 3 | 0.07 | 0.09 |
| 5 | 0.10 | 0.10 |
| 7 | 0.09 | 0.11 |
| 9 | 0.07 | 0.08 |
| 10 | 0.05 | 0.05 |
| 25 | 0.11 | 0.12 |

**TABLE B. Accelerated stability tests (72 hours). Percentage of total impurities.**

| **Batch** | **t** = **0** | **50°C/sat** |
|---|---|---|
| Azilect | 0.13 | 0.29 |
| 1 | 0.09 | 0.21 |
| 3 | 0.07 | 0.21 |
| 5 | 0.10 | 0.16 |
| 6 | 0.10 | 0.12 |
| 7 | 0.09 | 0.15 |
| 9 | 0.07 | 0.08 |
| 10 | 0.05 | 0.11 |
| 13 | 0.18 | 0.29 |
| 14 | 0.20 | 0.28 |
| 18 | 0.10 | 0.21 |
| 24 | 0.13 | 0.19 |
| 25 | 0.11 | 0.26 |

**TABLE C. Accelerated stability tests (72 hours). Percentage of total impurities.**

| **Batch** | **t=0** | **70°C** |
|---|---|---|
| Azilect | 0.13 | 0.49 |
| 2 | 0.08 | 0.40 |
| 4 | 0.14 | 0.46 |
| 7 | 0.09 | 0.36 |
| 8 | 0.25 | 0.35 |
| 9 | 0.07 | 0.50 |
| 10 | 0.05 | 0.27 |
| 11 | 0.19 | 0.24 |

**TABLE D. Accelerated ICH stability test for tablets packaged in aluminium/PVC blister packs. Percentage of total impurities.**

| 25°C and 60 % RH: | | | | |
|---|---|---|---|---|
| **Batch** | **t=0** | **15 days** | **30 days** | **60 days** |
| Azilect | 0.26 | 0.24 | 0.28 | 0.32 |
| 14 | 0.20 | 0.25 | 0.17 | 0.15 |
| 16 | 0.21 | 0.22 | 0.15 | 0.17 |
| 18 | 0.17 | 0.18 | 0.18 | 0.20 |
| 19 | 0.21 | 0.21 | 0.26 | 0.22 |

| 30°C and 65 % RH: | | | | |
|---|---|---|---|---|
| **Batch** | **t=0** | **15 days** | **30 days** | **60 days** |
| Azilect | 0.26 | 0.27 | 0.22 | 0.33 |
| 14 | 0.20 | 0.25 | 0.18 | 0.19 |
| 16 | 0.21 | 0.24 | 0.17 | 0.18 |
| 18 | 0.17 | 0.21 | 0.21 | 0.21 |
| 19 | 0.21 | 0.27 | 0.51 | |

| 40°C and 75 % RH: | | | | |
|---|---|---|---|---|
| **Batch** | **t=0** | **15 days** | **30 days** | **60 days** |
| Azilect | 0.26 | 0.31 | 0.35 | 0.53 |
| 14 | 0.20 | 0.27 | 0.30 | 0.52 |
| 16 | 0.21 | 0.28 | 0.25 | 0.41 |
| 18 | 0.17 | 0.24 | 0.35 | 0.25 |
| 19 | 0.21 | 0.31 | 0.42 | 0.49 |

**TABLE E. Accelerated ICH stability test for tablets packaged in aluminium/aluminium blister packs. Percentage of total impurities.**

| 25°C and 60 % RH: | | | | |
|---|---|---|---|---|
| **Batch** | **t=0** | **15 days** | **30 days** | **60 days** |
| Azilect | 0.26 | 0.24 | 0.28 | 0.32 |
| 14 | 0.20 | 0.25 | 0.17 | 0.15 |
| 16 | 0.21 | 0.22 | 0.15 | 0.17 |
| 18 | 0.17 | 0.18 | 0.18 | 0.20 |
| 19 | 0.21 | 0.21 | 0.26 | 0.22 |

| 30°C and 65 % RH: | | | | |
|---|---|---|---|---|
| **Batch** | **t=0** | **15 days** | **30 days** | **60 days** |
| Azilect | 0.26 | 0.27 | 0.22 | 0.33 |
| 14 | 0.20 | 0.22 | 0.16 | 0.20 |
| 16 | 0.21 | 0.24 | 0.30 | 0.19 |
| 18 | 0.17 | 0.21 | 0.24 | 0.33 |
| 19 | 0.21 | 0.27 | 0.24 | 0.33 |

| 40°C and 75 % RH: | | | | |
|---|---|---|---|---|
| **Batch** | **t=0** | **15 days** | **30 days** | **60 days** |
| Azilect | 0.26 | 0.31 | 0.35 | 0.53 |
| 14 | 0.20 | 0.29 | 0.28 | 0.52 |
| 16 | 0.21 | 0.29 | 0.33 | 0.30 |
| 18 | 0.17 | 0.28 | 0.30 | 0.55 |
| 19 | 0.21 | 0.36 | 0.34 | 0.33 |

### Example 29: Water content

The amount of water of the pharmaceutical compositions as herein disclosed was measured by loss on drying (LOD) using a Halogen Moisture Analyzer.

| Batch | % w/w water |
|---|---|
| 10 | 0.61 |
| 15 | 0.67 |
| 18 | 0.83 |
| 20 | 3.35 |
| 22 | 3.14 |

### Example 30: Particle size volume distribution

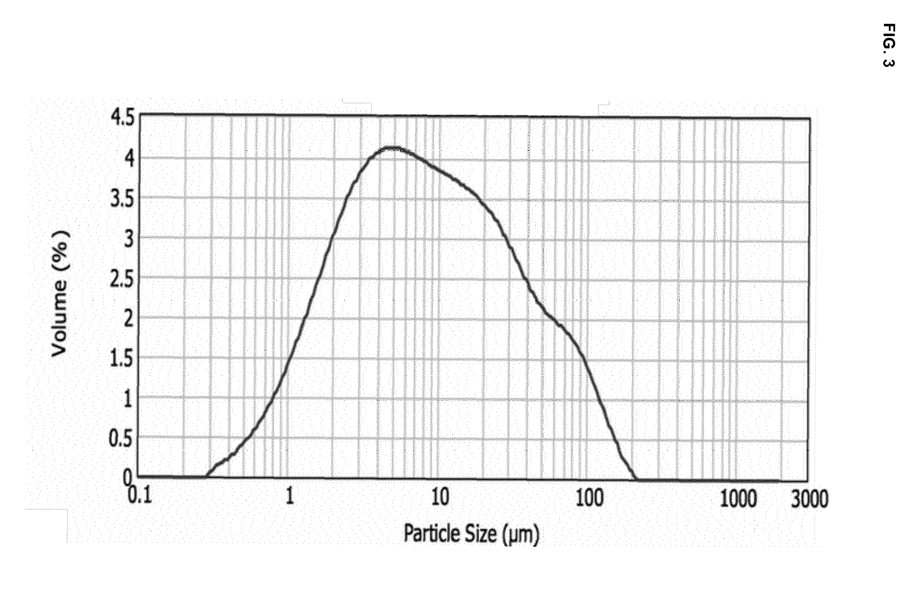

The particle size distribution of (*R*)-N-propargyl-1-aminoindan hemitartrate was analysed by laser diffraction spectroscopy using a Malvern Mastersizer 2000 particle size analyzer. The particle size distribution was D10 of 1.6 microns. D50 of 8.2 microns and D90 of 55.9 microns (Fig. 3).

### Example 31: Synthesis of (R)-N-propargyl-1-aminoindan hemitartrate

The process for the synthesis of (*R*)-N-propargyl-1-aminoindan hemitartrate is described in WO2010059913. The (*R*)-N-propargyl-1-aminoindan hemitartrate obtained by this process was characterised by X-Ray Powder Diffraction and is crystalline form GH of (*R*)-N-propargyl-1-aminoindan hemitartrate. The *(R)*-N-propargyl-1-aminoindan hemitartrate obtained by this process shows no agglomerations.

Also. WO2010007181 describes processes for the synthesis of (*R*)-N-propargyl-1-aminoindan tartrate and (*R*)-N-propargyl-1-aminoindan hemitartrate among other (*R*)-N-propargyl-1-aminoindan salts.

### Example 32: X-Ray Powder Diffraction

X-Ray Powder Diffraction was performed in a Bruker D8 Advance diffractometer with a θ:2θ configuration and Bragg-Brentano geometry with a copper anode tube. The difractograma is obtained for 2θ angles ranging from 3° to 70° with a step of 0.03° each second. The tube set-up is 40 kV and 30 mA, incident-beam divergence-limiting slit 12 mm, static sample, diffracted-beam receiving slit 0.2 mm and Nickel filter.

Crystalline form GH of (*R*)-N-propargyl-1-aminoindan hemitartrate of figure 2 has the following peaks:

| Angle 2θ | Intensity |
|---|---|
| 6.7 | 12,035 |
| 8.2 | 1,343 |
| 10.3 | 680 |
| 10.7 | 266 |
| 12.6 | 8,510 |
| 13.3 | 2,988 |
| 14.7 | 1,524 |
| 15.2 | 2,004 |
| 16.4 | 8,471 |
| 17.3 | 1,634 |
| 18.5 | 1,684 |
| 19.1 | 1,314 |
| 20.4 | 4,470 |
| 21.3 | 1,510 |
| 21.9 | 3,032 |
| 23.0 | 7,818 |
| 24.4 | 2,146 |
| 25.0 | 2,948 |
| 25.5 | 1,322 |
| 26.8 | 3,385 |
| 28.0 | 3,787 |
| 29.6 | 2,150 |

Unless otherwise indicated, all the analysis methods are carried out according to the European Pharmacopoeia 7th edition.

## Claims

1. A stable pharmaceutical composition in the form of immediate release tablets comprising (*R*)-N-propargyl-1-aminoindan hemitartrate, preferably for use in the treatment of Parkinson's disease.

2. The pharmaceutical composition according to the preceding claim comprising between a 0.3 and a 1.5 % by weight of (*R*)-N-propargyl-1-aminoindan hemitartrate in respect of the total amount of the pharmaceutical composition.

3. The pharmaceutical composition according to any one of the preceding claims, wherein the pharmaceutical composition further comprises at least an acid, preferably the total amount of acid or acids present in the pharmaceutical composition ranges from 0.3 to 3.5 % by weight in respect of the total amount of the pharmaceutical composition, the pharmaceutical composition comprises at least an acid selected from citric acid and tartaric acid and/or the pharmaceutical composition comprises tartaric acid.

4. The pharmaceutical composition according to any one of the preceding claims, wherein the (*R*)-N-propargyl-1-aminoindan hemitartrate is in crystalline form or the pharmaceutical composition comprises crystalline form GH of (*R*)-N-propargyl-1-aminoindan hemitartrate **characterised by** at least X-ray powder diffraction peaks 6.7, 12.6 and 16.4° 2θ (±0.2° 2θ), and/or the D50 of (*R*)-N-propargyl-1-aminoindan hemitartrate is between 4 to 39 microns as measured by laser diffraction spectroscopy and/or the D90 of (*R*)-N-propargyl-1-aminoindan hemitartrate is less than 159 microns as measured by laser diffraction spectroscopy.

5. The pharmaceutical composition according to any one of the preceding claims, wherein the pharmaceutical composition comprises at least a filler, preferably: the total amount of filler or fillers present in the pharmaceutical composition ranges from 25 % to 95 % by weight in respect of the total amount of the pharmaceutical composition; the total amount of filler or fillers present in the pharmaceutical composition ranges from 45 % to 90 % by weight in respect of the total amount of the pharmaceutical composition; and/or the pharmaceutical composition comprises at least a filler, selected from microcrystalline cellulose, mannitol, trehalose, and mixtures thereof, more preferably the pharmaceutical composition comprises trehalose.

6. The pharmaceutical composition according to any one of the preceding claims, wherein the pharmaceutical composition comprises at least a lubricant, preferably the total amount of lubricant or lubricants present in the pharmaceutical composition ranges from 0.5 to 5 % by weight in respect of the total amount of the pharmaceutical composition and/or the pharmaceutical composition comprises at least a lubricant selected from magnesium stearate, stearic acid, sodium stearyl fumarate or mixtures thereof, more preferably the pharmaceutical composition comprises stearic acid.

7. The pharmaceutical composition according to any one of the preceding claims, wherein the pharmaceutical composition comprises at least a disintegrant, preferably the total amount of disintegrant or disintegrants present in the pharmaceutical composition ranges from 5 to 25 % by weight in respect of the total amount of the pharmaceutical composition and/or the pharmaceutical composition comprises at least a disintegrant selected from starch, pregelatinized starch, and low-substituted hydroxypropyl cellulose, more preferably the pharmaceutical composition comprises pregelatinized starch.

8. The pharmaceutical composition according to any one of the preceding claims, wherein the pharmaceutical composition comprises between 0.74 and 2.18 mg of (*R*)-N-propargyl-1-aminoindan hemitartrate by unit dose, preferably the pharmaceutical composition comprises about 1.44 mg of (*R*)-N-propargyl-1-aminoindan hemitartrate by unit dose.

9. The pharmaceutical composition according to the preceding claim, wherein the pharmaceutical compositions is manufactured by wet granulation techniques or dry mix techniques, preferably the pharmaceutical compositions is manufactured by direct compression.

10. (*R*)-N-propargyl-1-aminoindan hemitartrate crystalline form GH having at least X-ray powder diffraction peaks 6.7, 12.6 and 16.4° 2θ (±0.2° 2θ), preferably having at least X-ray powder diffraction peaks 6.7, 12.6, 13.3, 16.4, 20.4 and 23.0° 2θ (±0.2° 2θ) or use of said (*R*)-N-propargyl-1-aminoindan hemitartrate for the manufacture of immediate release tablets comprising (*R*)-N-propargyl-1-aminoindan hemitartrate, and/or use of said (*R*)-N-propargyl-1-aminoindan hemitartrate for a process for the manufacture of immediate release tablets comprising *(R)*-N-propargyl-1-aminoindan hemitartrate.

11. A pharmaceutical batch comprising at least 20,000 units, preferably comprising at least 50,000 units, more preferably comprising at least 100,000 units of the pharmaceutical composition as defined in any one of claims 1 to 9, wherein the *(R)-*N-propargyl-1-aminoindan hemitartrate content is uniform and/or the tablets are packaged in a blister pack of aluminium/PVC or aluminium/aluminium.

12. A process for the manufacture of the pharmaceutical composition as defined in claims 1 to 9 or the pharmaceutical batch as defined in claim 11, wherein the process comprises the following steps:
i) sieving when appropriate the ingredients through a 500 micron sieve;
ii) mixing (*R*)-N-propargyl-1-aminoindan hemitartrate with at least one pharmaceutically acceptable excipient, preferably a filler, preferably at least 10 % of the total weight of said filler;
iii) further, and when appropriate, mixing the mixture obtained in step (ii) with further pharmaceutically acceptable excipients;
iv) optionally, mixing the mixture obtained in step (iii) or (ii) with at least one lubricant; and
v) compressing the mix into tablets.

13. A process for the manufacture of a stable pharmaceutical composition comprising (*R*)-N-propargyl-1-aminoindan hemitartrate in the form of an immediate release tablet or for the manufacture of a pharmaceutical batch of said pharmaceutical composition, wherein the process comprises the following steps:
i) preparing a test pharmaceutical composition comprising (*R*)-N-propargyl-1-aminoindan hemitartrate in the form of a tablet;
ii) checking the stability and dissolution profile of the test pharmaceutical composition of step (i), preferably the stability after at least one day at 40°C and 75 % relative humidity; and
iii) manufacturing a pharmaceutical composition comprising *(R)*-N-propargyl-1-aminoindan hemitartrate in the form of an immediate release tablet or a pharmaceutical batch of said pharmaceutical composition, by the same process used to prepare the test pharmaceutical composition in step (i) provided that the test composition is stable and has an immediate release dissolution profile; and
iv) optionally, packaging the stable pharmaceutical composition or the pharmaceutical batch manufactured in step (iii), preferably in blister packs or in bottles.

14. A pharmaceutical batch validated by a process comprising the following steps:
i) manufacturing the pharmaceutical batch;
ii) checking the uniformity of the (*R*)-N-propargyl-1-aminoindan hemitartrate content; and
iii) validating the batch only if the content is uniform.

15. A packaged pharmaceutical composition, wherein said packaged pharmaceutical composition is: a blister pack comprising the pharmaceutical composition as defined in any one of claims 1 to 9, or a bottle containing at least 10 tablets as defined in any one of the claims 1 to 9, or a cardboard box with a patient information leaflet comprising at least one of said blister pack or said bottle.
